(19) Europäisches Patentamt
European Patent Office
Office européen des brevets

(11) Publication number: 0 188 538
B1

(12) **EUROPEAN PATENT SPECIFICATION**

(45) Date of publication of patent specification: **11.10.89**

(51) Int. Cl.$^4$: **A 61 K 31/445**

(21) Application number: **85903582.6**

(22) Date of filing: **01.07.85**

(86) International application number: **PCT/US85/01246**

(87) International publication number: **WO 86/00806 13.02.86 Gazette 86/04**

# (54) TRANSDERMAL DELIVERY OF AZATADINE.

(30) Priority: **23.07.84 US 633541**

(43) Date of publication of application: **30.07.86 Bulletin 86/31**

(45) Publication of the grant of the patent: **11.10.89 Bulletin 89/41**

(84) Designated Contracting States: **AT BE CH DE FR GB IT LI LU NL SE**

(56) References cited:
**DE-A-1 795 745**
**US-A-3 435 114**

(73) Proprietor: **SCHERING CORPORATION**
**2000 Galloping Hill Road**
**Kenilworth New Jersey 07033 (US)**

(72) Inventor: **SEQUEIRA, Joel, August**
**18 Stuyvesant Oval**
**New York, NY 10009 (US)**

(74) Representative: **von Kreisler, Alek, Dipl.-Chem. et al**
**Deichmannhaus am Hauptbahnhof**
**D-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 188 538 B1

**Description**

This invention relates to the use of azatadine or a salt thereof in pharmaceutical compositions suitable for transdermal delivery, to such transdermal compositions containing azatadine or its salt and to the treatment of allergic reactions by transdermal application of azatadine or a salt thereof.

In recent years, various drug delivery systems have been developed which provide sustained release therapy via a sub-dermal insert. Systems have been disclosed which also provide drug delivery systems suitable for transdermal drug administration.

U.S. Pat. No. 4,336,243 discloses a transdermal delivery pad for nitroglycerin administration, nitroglycerin dispersion and transport.

The use of transdermal drug delivery systems produces more controlled blood levels, lower frequency of dosing and enhanced patient compliance. Further, the transdermal delivery of an antihistamine to treat various allergic reactions is most desirable for reasons of convenience and effectiveness.

Unfortunately, however, until now no antihistamine seemed to possess the properties necessary in order to be effective in a transdermal drug delivery system. These properties are high potency, proper physico-chemical characteristics, good dermal penetration and, lack of dermal irritation and/or sensitization.

Quite unexpectedly, azatadine has been found to possess all of the above described properties which are necessary for being effective in a transdermal drug delivery system. Frequently, in order to obtain effective dermal penetration of drugs in a transdermal delivery system, a promoter such as dimethyl sulfoxide is used. It is unexpected that azatadine's transdermal effectiveness is not dependent on the use of such a promoter.

Azatadine, 6,11-diydro-11-(1-methyl-4-piperidylidene)-5*H*-benzo[5,6]cyclohepta[1,2-b]pyridine

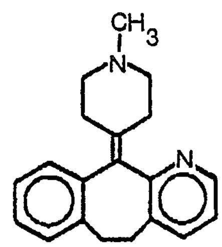

is known as a potent antihistamine which is recognised as clinically effective for treating various allergic reactions but until now it has only been applied by the oral route.

The invention sought to be patented in its method aspect is a pharmaceutical method for treating allergic reactions in a mammal which comprises the transdermal application of an effective amount of azatadine combined with a pharmaceutically acceptable transdermal carrier.

The invention sought to be patented in a transdermally acceptable pharmaceutical dosage formulation and or composition, comprises an effective amount of azatadine in combination with a pharmaceutically acceptable transdermal carrier. Preferably, the transdermally acceptable composition is utilized to prepare a "reservoir type" or "matrix type" patch which is applied to the skin and worn for a specific period of time to permit the penetration of a desired amount of azatadine through the skin. Most preferably, the patch of the invention will be worn for a period of about 24 hours and provide a total daily dosage of about 0.5 mg to about 1.5 mg of azatadine. The patch may then be replaced if necessary with a fresh patch, thereby providing a constant blood level of azatadine to the patient in need thereof.

Azatadine and the preparation thereof are described in U.S. Patent 3,326,924. Pharmaceutically acceptable salts of azatadine such as the maleate, sulfate, succinate and acetate salts may also be prepared as described therein and these are equivalent to azatadine for purposes of the invention. A preferred pharmaceutically acceptable salt of azatadine is the maleate salt.

Azatadine, is an orally acceptable antihistamine currently approved for sale in the United States and other countries of the world for use in the treatment of allergic disorders such as urticaria, seasonal rhinitis and pollen sensitivity.

In an attempt to find a transdermally effective antihistamine, various known antihistamines were evaluated for their dermal irritation and dermal penetration properties. The results of these evaluations are in Table 1. The irritation results are reported as either irritating (+) or non-irritating (−), and the penetration results are reported as either good (+) or poor (−). Some antihistamines listed were not tested for in vitro dermal penetration and these are indicated as.

TABLE 1

Transdermal Application of Antihistamines

| Compound | Rabbit Dermal Irritation a* | *In Vitro* Dermal Penetration |
|---|---|---|
| Chlorpheniramine base (patch) | (+) | (+) |
| Chlorpheniramine maleate (20%) | (−) | (−) |
| Chlorpheniramine succinate (20%) | (−) | (−) |
| Chlorpheniramine sulfate (20%) | (−) | (−) |
| Chlorpheniramine acetate (20%) | (+) | (+) |
| Chlorpheniramine palmitate (20%) | (+) | (+) |
| Pheniramine base (20%) | (−) | — |
| Dexbrompheniramine (10%) | (+) | — |
| Dexchlorpheniramine base | (+) | — |
| Azatadine | (−) | (+) |
| Doxylamine succinate | (+) | — |
| Diphenhydramine HCl | (+) | — |
| Triprolidine HCl | (+) | — |
| Diphenylpyraline HCl | (+) | — |
| Cyprhoheptadine HCl | (+) | — |
| Promethazine HCl | (+) | — |
| Carbinoxamine maleate | (+) | — |
| Dimethindine maleate | (+) | — |

a* = Results of a 24/72 hour (no irritation seen for the first 24 hours) dermal irritation screen in rabbits with intact skin.

Screening of Antihistamine for Dermal Irritation

Eight young adult New Zealand white rabbits were assigned to two groups. The rabbits were housed in stainless steel cages and maintained under standard laboratory conditions. Each cage was identified with a color coded label indicating the test substance, rabbit number, sex, and study number. Food and water were supplied *ad libitum*. On the day of dosing, the back of each rabbit was clipped free of hair. The test substance, a 20% azatadine cream formulation, or a placebo cream was instilled into two Hill Top Chambers and affixed on on each side of the midline of the back. To prevent removal, the chambers were covered with an orthopedic stockinette and secured by a canvas coat. Signs of dermal irritation graded on the basis of criteria presented in Draize, J. H.; Dermal Toxicology, An Appraisal Of The Safety And Chemical In Food Drugs & Cosmetics, The Association of Food & Drug Officials of the United States 1959 pp 46—59, were recorded after removal of the chambers at 24 and 72 hours.

Screening of Antihistamines for Dermal Penetration

The skin penetration assembly used was similar to that described by Franz (J. Invest. Derm., 64:190, 1975). Excised defatted human skin was stretched across a reservoir containing a phosphate buffer solution (pH 7.4, 0.02M) in direct contact with the dermal side of the skin. The temperature of this buffer solution was maintained at 37± 0.5°C by circulating water at the appropriate temperature through a jacket which surrounds each assembly. Freshly made preparation was applied to the statum corneum surface. The

buffer solution was removed in its entirety and replaced with fresh solution at various time intervals and assayed for azatadine content.

Several antihistamines were eliminated from consideration as effective transdermal drugs because they were shown to be dermal irritants. Another group of antihistamines, though they showed no dermal irritation, did not show dermal penetration, the latter property being an essential requirement for the effectiveness of a transdermal drug.

The results given in Table 1 reveal that usually antihistamines which give positive results for dermal penetration are unacceptable for transdermal applications because of their dermal irritant properties. It is surprising to find that after numerous antihistamines were tested, there was only one antihistamine, azatadine, that had good dermal penetration and no dermal irritation and/or sensitization. Thus, the results in Table 1 show that of the compounds tested for transdermal application, only azatadine possess the characteristics necessary for transdermal use.

The use of a particular transdermal formulation is not critical to the practice of the invention. Thus, the invention contemplates the use of any formulation, including those not yet discovered or fully characterized, so long as said transdermal dosage form can be utilized to transdermally deliver the desirable amount of azatadine.

It is of interest to the practice of the present invention that the totally daily dosage (through the skin) of azatadine which is administered by the transdermal formulation may be less than the currently recommended clinical daily administered dose by the oral routes. Morevoer, it is anticipated that upon implementive of the invention the azatadine blood levels will be more consistent and controlled than those obtained upon oral administration of the drug. However, this is not a requirement of the invention. This feature is anticipated in view of clinical experience with other transdermal drugs. Thus, the total daily transdermal dosage of azatadine when administered in a patch is expected to be from about 0.2 mg to about 5.0 mg, with 0.5 mg to about 1.5 mg being preferred. The particular dosage may be varied depending on the size and age of the patient and may also depend upon the severity of the condition being treated. Such dosage modification is within the skill of the clinical arts. The utilization of this new dosage form and its prescribed regimen will provide the recognized clinical efficacy of azatadine, having the advantages described above. Other frequencies of dosage application are anticipated, for example, a once every 3 day frequency or a once every 7 day frequency. Although a once a day dosage regimen may be preferred, it is not intended that the invention be limited to any particular regimen.

Table 2 contains *in vitro* skin diffusion flux results obtained using human cadaver skin in a Franz diffusion cell, for a 1 $cm^2$ area, flux is defined as the amount of drug that traverses skin over time for a specified area.

TABLE 2

Accumulative Azatadine (mg/per $cm^2$)

| Formulation[a] | 20 Hr | 45 Hr | 72 Hr |
|---|---|---|---|
| Ointment A | 2.10 | 7.54 | 13.83 |
| Ointment B | 3.24 | 10.16 | 17.38 |
| Cream | 3.01 | 13.69 | 27.41 |
| Gel | 2.38 | 7.11 | 11.79 |

[a]Prepared in Examples 1—4, respectively

This data (Table 2) indicates that azatadine traverses human skin in amounts which are clinically effective. The transdermal dose for clinical effectiveness is expected to be 1 mg/day. Azatadine flux rates of greater than 1 mg day (the preferred daily clinical dosage) were achieved within 20 hours for all of the formulation evaluated (Table 2). Those flux rates achieved a steady-state level between 20—45 hours post patch application. Moreover, these high flux rates were achieved without the use of promotants (eg dimethyl sulfoxide).

The following examples illustrate formulations of azatadine that show good dermal penetration.

Example 1

| Ointment A | mg/g |
|---|---|
| Azatadine | 200 |
| White Petrolatum | 800 |

Example 2

| Ointment B | mg/g |
|---|---|
| Azatadine | 200 |
| Propylene glycol | 200 |
| White Petrolatum | 600 |

Example 3

| Cream | mg/g |
|---|---|
| Azatadine | 200 |
| Mineral Oil | 48 |
| White Petrolatum | 120 |
| Cetostearyl Alcohol | 57.6 |
| Polyethylene glycol 1000 monocetylether | 18.0 |
| Propylene glycol | 80 |
| Water | 476.4 |

Example 4

| Gel | mg/g |
|---|---|
| Azatadine | 200 |
| Pluronic F—127® | 250 |
| Ethanol | 200 |
| Water | 350 |

Example 5

| Patch | mg/g |
|---|---|
| Azatadine | 100.00 |
| Mineral Oil | 54.0 |
| White Petrolatum | 135.0 |
| Ceteryl Alcohol | 65.0 |
| Ceteth 20 | 20.0 |
| Propylene Glycol | 100.0 |
| Water q.s. ad | 1.0 g |

The formulations of examples 1—5 can be packaged to produce a "reservoir type" transdermal patch with or without a rate-limiting patch membrane. The size of the patch and or the rate limiting membrane can be chosen to deliver the transdermal flux rates desired. Alternatively, azatadine and/or its salts can be

formulated into a "matrix-type" transdermal patch as in examples 5 and 6. Drug Delivery Systems Characteristics and Biomedical Application, R.L. Juliano, ed., Oxford University Press, N.Y. (1980); and Controlled Drug Delivery Vol. I Basic Concepts, Stephen D. Bruck (1983) describe the theory and application of methods useful for transdermal delivery systems. The relevant teachings of these texts are herein incorporated by reference. The drug-matrix could be formed utilizing various polymers, e.g. silicone, polyvinyl alcohol, polyvinyl chloride-vinyl acetate co-polymer. The "drug matrix" may then be packaged into an appropriate transdermal patch.

Example 6

| Patch | mg/g |
|---|---|
| Azatadine | 200 |
| silicone polymer | 800 |

Example 7

| Patch | mg/g |
|---|---|
| Azatadine | 300 |
| Polyvinyl chloride vinyl acetate co-polymer | 700 |

The invention also contemplates a package which contains a specific number of transdermal patches that may be utilized to complete a specified course of treatment. For example, a package containing 7, 24 hours patches would be utilized to complete a one week course of therapy.

**Claims for the Contracting States: BE CH DE FR GB IT LI LU NL SE**

1. Transdermally effective topical anti-allergic pharmaceutical compositions comprising, as an active ingredient, azatadine or a salt thereof in combination with pharmaceutically acceptable transdermal carriers.
2. Composition according to claim 1 which is formulated as an ointment.
3. An ointment according to claim 2 which comprises 200 mg of azatadine and 800 mg of white petrolatum.
4. An ointment according to claim 2 which comprises 200 mg of azatadine, 200 mg of propylene glycol and 600 mg of white petrolatum.
5. Composition according to claim 1 which is formulated as a cream.
6. A cream according to claim 5 which comprises 25—250 mg azatadine, 25—100 mg mineral oil, 50—200 mg white petrolatum, 30—100 mg cetearyl alcohol, 10—50 ceteth 20, 50—200 mg propylene glycol and water, the quantities of the ingredients being selected so that it gives a total of 1 g.
7. A cream according to claim 6 which comprises 100 mg azatadine, 54 mg mineral oil, 135 mg white petrolatum, 65 mg cetearyl alcohol, 20 mg ceteth 20, 100 mg propylene glycol and water to make a total weight of 1 g.
8. A cream according to claim 5 which comprises 200 mg of azatadine, 48 mg of mineral oil, 120 mg of white petrolatum, 57.6 mg of cetostearyl alcohol, 18 mg of polyethylene glycol 1000 monocetyl ether, 80 mg of propylene glycol and 476.4 mg of water.
9. Composition according to claim 1 which is formulated as a gel.
10. Gel defined according to claim 8 which comprises 200 mg of azatadine, 250 mg of pluronic F—127®, 200 mg of ethanol and 350 mg of water.
11. Composition according to claim 1 in the form of a patch.

**Claims for the Contracting State: AT**

1. Process for preparing transdermally effective topical anti-allergic pharmaceutical compositions which comprises mixing, as an active ingredient, azatadine or a salt thereof with pharmaceutically acceptable transdermal carriers.
2. Process according to claim 1 wherein the composition is formulated as an ointment.
3. Process according to claim 2 wherein the ointment comprises 200 mg of azatadine and 800 mg of white petrolatum.
4. Process according to claim 2 wherein the ointment comprises 200 mg of azatadine, 200 mg of propylene glycol and 600 mg of white petrolatum.
5. Process according to claim 1 wherein the composition is formulated as a cream.
6. Process according to claim 5 wherein the cream comprises 25—250 mg azatadine, 25—100 mg mineral oil, 50—200 mg white petrolatum, 30—100 mg cetearyl alcohol, 10—50 mg ceteth 20, 50—200 mg

propylene glycol and water, the quantities of the ingredients being selected so that it gives a total of 1 g.

7. Process according to claim 6 wherein the cream comprises 100 mg azatadine, 54 mg mineral oil, 135 mg white petrolatum, 65 mg cetearyl alcohol, 20 mg ceteth 20, 100 mg propylene glycol and water to make a total weight of 1 g.

8. Process according to claim 5 wherein the cream comprises 200 mg of azatadine, 48 mg of mineral oil, 120 mg of white petrolatum, 57.6 mg of cetostearyl alcohol, 18 mg of polyethylene glycol 1000 monocetyl ether, 80 mg of propylene glycol and 476.4 mg of water.

9. Process according to claim 1 wherein the composition is formulated as a gel.

10. Process defined according to claim 8 wherein the gel comprises 200 mg of azetadine, 250 mg of pluronic F—127®, 200 mg of ethanol and 350 mg of water.

11. Process according to claim 1 wherein the composition is in the form of a patch.

**Patentansprüche für die Vertragsstaaten: BE CH DE FR GB IT LI LU NL SE**

1. Transdermal wirksame, topische antiallergische pharmazeutische Zusammensetzungen, umfassend als wirkstoff Azatadin oder ein Salz desselben in Kombination mit pharmazeutisch annehmbaren transdermalen Trägern.

2. Zusammensetzung nach Anspruch 1, die als Salbe formuliert ist.

3. Salbe nach Anspruch 2, die 200 mg Azatadin und 800 mg wießes Petrolatum umfaßt.

4. Salbe nach Anspruch 2, die 200 mg Azatadin, 200 mg Propylenglycol und 600 mg weißes Petrolatum umfaßt.

5. Zusammensetzung nach Anspruch 1, die als Creme formuliert ist.

6. Creme nach Anspruch 5, die 25—250 mg Azatadin, 25—100 mg Mineralöl, 50—200 mg weißes Petrolatum, 30—100 mg Cetarylalkohol, 10—50 mg Ceteth 20, 50—200 mg Propylenglycol und Wasser umfaßt, wobei die Mengen der Bestandteile so gewählt sind, daß sie insgesamt 1 g ergeben.

7. Creme nach Anspruch 6, die 100 mg Azatadin, 54 mg Mineralöl, 135 mg weißes Petrolatum, 65 mg Ceetarylalkohol, 20 mg Ceteth 20, 100 mg Propylenglycol und Wasser bis zum Gesamtgewicht von 1 g umfaßt.

8. Creme nach Anspruch 5, die 200 mg Azatadin, 48 mg Mineralöl, 120 mg weißes Petrolatum, 57,6 mg Cetostearylalkohol, 18 mg Polyethylenglycol 1000-monocetylether, 80 mg Propylenglycol und 476,4 mg Wasser umfaßt.

9. Zusammensetzung nach Anspruch 1, die als Gel formuliert ist.

10. Gel nach Anspruch 9, das 200 mg Azatadin, 250 mg Pluronic F—127®, 200 mg Ethanol und 350 mg Wasser umfaßt.

11. Zusammensetzung nach Anspruch 1 in Form eines Pflasters.

**Patentansprüche für den Vertragsstaat: AT**

1. Verfahren zur Herstellung transdermal wirksamer, topischer antiallergischer pharmazeutischer Zusammensetzungen, umfassend das Vermischen von Azatadin oder einem Salz desselben als Wirkstoff mit pharmazeutisch annehmbaren transdermalen Trägern.

2. Verfahren nach Anspruch 1, worin die Zusammensetzung als Salbe formuliert ist.

3. Verfahren nach Anspruch 2, worin die Salbe 200 mg Azatadin und 800 mg weißes Petrolatum umfaßt.

4. Verfahren nach Anspruch 2, worin die Salbe 200 mg Azatadin, 200 mg Propylenglycol und 600 mg weißes Petrolatum umfaßt.

5. Verfahren nach Anspruch 1, worin die Zusammensetzung als Creme formuliert ist.

6. Verfahren nach Anspruch 5, worin die Creme 25—250 mg Azatadin, 25—100 mg Mineralöl, 50—200 mg weißes Petrolatum, 30—100 mg Ceetarylalkohol, 10—50 mg Ceteth 20, 50—200 mg Propylenglycol und Wasser umfaßt, wobei die Mengen der Bestandteile so gewählt sind, daß sie insgesamt 1 g ergeben.

7. Verfahren nach Anspruch 6, worin die Creme 100 mg Azatadin, 54 mg Mineralöl, 135 mg weißes Petrolatum, 65 mg Ceetarylalkohol, 20 mg Ceteth 20, 100 mg Propylenglycol und Wasser bis zum Gesamtgewicht von 1 g umfaßt.

8. Verfahren nach Anspruch 5, worin die Creme 200 mg Azatadin, 48 mg Mineralöl, 120 mg weißes Petrolatum, 57,6 mg Cetostearylalkohol, 18 mg Polyethylenglycol 1000-monocetylether, 80 mg Propylenglcol und 476,4 mg Wasser umfaßt.

9. Verfahren nach Anspruch 1, worin die Zusammensetzung als Gel formuliert ist.

10. Verfahren nach Anspruch 9, worin das Gel 200 mg Azatadin, 250 mg Pluronic F—127®, 200 mg Ethanol und 350 mg Wasser umfaßt.

11. Verfahren nach Anspruch 1, worin die Zusammensetzung in Form eines Pflasters vorliegt.

**Revendications pour les Etats contractants: BE CH DE FR GB IT LI LU NL SE**

1. Compositions pharmaceutiques topiques anti-allergiques transdermiquement efficaces, comprenant, comme ingrédient actif, de l'azatadine ou de son sel en combinaison avec des véhicules

transdermiques pharmaceutiquement acceptables.

2. Composition selon la revendication 1 qui est formulée en un onguent.

3. Onguent selon la revendication 2 qui contient 200 mg d'azatadine et 800 mg de pétrolatum blanc.

4. Onguent selon la revendication 2 qui contient 200 mg d'azatadine, 200 mg de propylène glycol et 600 mg de pétrolatum blanc.

5. Composition selon la revendication 1 qui est formulée en une crème.

6. Crème selon la revendication 5 qui contient 25—250 mg d'azatadine, 25—100 mg d'une huile minérale, 50—200 mg de pétrolatum blanc, 30—100 mg d'alcool cétéarylique, 15—50 mg de ceteth 20, 50—200 mg de propylène glycol et de l'eau, les quantités des ingrédients étant choisies de manière à donner un total de 1 g.

7. Crème selon la revendication 6 qui contient 100 mg d'azatadine, 54 mg d'huile minérale, 135 mg de pétrolatum blanc, 65 mg d'alcool cétéarylique, 20 mg de ceteth 20, 100 mg de propylène glycol et de l'eau pour obtenir un poids total de 1 g.

8. Crème selon la revendication 5 qui contient 200 mg d'azatadine, 48 mg d'huile minérale, 120 mg de pétrolatum blanc, 57,6 mg d'alcool cétostéarylique, 18 mg de polyéthylène glycol 100 monocétyléther, 80 mg de propylène glycol et 476,4 mg d'eau.

9. Composition selon la revendication 1 qui est formulée en un gel.

10. Gel selon la revendication 8 qui contient 200 mg d'azatadine, 250 mg de pluronic F—127®, 200 mg d'éthanol et 350 mg d'eau.

11. Composition selon la revendication 1 sous la forme d'une emplâtre.

**Revendications pour l'Etat contractant: AT**

1. Procédé de préparation de compositions pharmaceutiques topiques anti-allergiques transdermiquement efficaces qui comprend le mélange, comme ingrédient actif, d'azatadine ou de son sel avec des véhicules transdermiques pharmaceutiquement acceptables.

2. Procédé selon la revendication 1 où la composition est formulée en un onguent.

3. Procédé selon la revendication 2 où l'onguent contient 200 mg d'azatadine et 800 mg de pétrolatum blanc.

4. Procédé selon la revendication 2 où l'onguent contient 200 mg d'azatadine, 200 mg de propylène glycol et 600 mg de pétrolatum blanc.

5. Procédé selon la revendication 1 où la composition est formulée en une crème.

6. Procédé selon la revendication 5 où la crème contient 25—250 mg d'azatadine, 25—100 mg d'une huile minérale, 50—200 mg de pétrolatum blanc, 300—100 mg d'alcool cétéarylique, 15—50 mg de ceteth 20, 50—200 mg de propylène glycol et de l'eau, les quantités des ingrédients étant choisies de manière à donner un total de 1 g.

7. Procédé selon la revendication 6 où la crème contient 100 mg d'azatadine, 54 mg d'huile minérale, 135 mg de pétrolatum blanc, 65 mg d'alcool cétéarylique, 20 mg de ceteth 20, 100 mg de proylène glycol et de l'eau pour obtenir un poids total de 1 g.

8. Procédé selon la revendication 5 où la crème contient 200 mg d'azatadine, 48 mg d'huile minérale, 120 mg de pétrolatum blanc, 57,6 mg d'alcool cétostéarylique, 18 mg de polyéthylène glycol 1000 monocétyléther, 80 mg de propylène glycol et 476,4 mg d'eau.

9. Procédé selon la revendication 1 où la composition est formulée en un gel.

10. Procédé selon la revendication 8 où le gel contient 200 mg d'azatadine, 250 mg de pluronic F—127®, 200 mg d'éthanol et 350 mg d'eau.

11. Procédé selon la revendication 1 où la composition a la forme d'une emplâtre.